# EUROPEAN PATENT SPECIFICATION

(11) **EP 2 684 869 B1**
(45) Date of publication and mention of the grant of the patent: **28.10.2015**
(21) Application number: 12755385.7
(22) Date of filing: 17.02.2012
(51) Int. Cl.: C07C 323/52, C07C 319/06

(54) **PREPARATION OF 3-MERCAPTOPROPIONATES**
HERSTELLUNG VON 3-MERCAPTOPROPIONATEN
PRÉPARATION DE 3-MERCAPTOPROPIONATES

(30) Priority: 08.03.2011 CN 201110059175
(43) Date of publication of application: 15.01.2014
(73) Proprietor: Beijing Tianqing Chemicals Co., Ltd., Beijing 101200 (CN)
(72) Inventor: XIAO, Haihuan, Pinggu District Beijing 101200 (CN); ZHANG, Xiaolin, Pinggu District Beijing 101200 (CN); JIN, Yuechun, Pinggu District Beijing 101200 (CN)
(74) Representative: Jansen, Cornelis Marinus
(86) International application number: PCT/CN2012/071280
(87) International publication number: WO 2012/119507

(56) References cited:
- DE-A1- 3 614 065
- JP-A- 6 298 725
- JP-A- 61 254 555
- US-A- 4 052 440
- US-A- 5 157 147
- CECIL ET AL: "A kinetic study of the reactions on some disulphides with sodium sulphite.", BIOCHEMICAL JOURNAL, vol. 60, no. 3, 1 July 1955 (1955-07-01), pages 496-506, XP55123492, ISSN: 0264-6021
- CECIL, R. ET AL.: 'A Kinetic Study of the Reactions on some Disulphides with Sodium Sulphite' BIOCH. vol. 60, no. 3, 1955, pages 496 - 506, XP055123492

## Description

### FIELD OF THE INVENTION

The present application relates to a process for preparing 3-mercapto propionates.

### BACKGROUND OF THE INVENTION

3-mercapto propionates are important chemical raw materials. For example, methyl 3-mercapto propionate is an intermediate for preparing isothiazolinone, and is also one of raw materials for preparing the fine chemical 4-chloro-7-methyl thieno[3,2-D]pyrimidine. A known process for preparing such compounds as for example described in DE 3614 065 A1 comprises reacting hydrogen sulfide with methyl acrylate under certain conditions. However, this process disclosed in the prior art generally suffers from lower yield. In addition, the above-mentioned reaction generally is carried out in an autoclave, which leads to more stringent requirements on apparatus. In addition, it is dangerous to introduce the gas into a reaction system under higher pressure, and moreover reaction procedures are complicated and difficultly performed in industrial scale.

### SUMMARY OF THE INVENTION

In one aspect, the present application provides a process for preparing 3-mercapto propionates, comprising adding Na₂SO₃ during a reaction of H₂S with S₂R₂, wherein R= -CH₂CH₂COOR¹, R¹ is C₁₋₈ alkyl, aryl C₁₋₈ alkyl or C₃₋₈ cycloalkyl.

### DESCRIPTION OF THE DRAWINGS

Figure 1 shows variation of contents of main reactants and products with time when Na₂SO₃ is added.
Figure 2 shows variation of a content of target product with a molar ratio of reactants in one embodiment of the present application.
Figure 3 shows variation of a content of target product with reaction temperature in another embodiment of the present application.
Figure 4 shows variation of contents of main reactants and products with time when Na₂SO₃ is not added.

### DETAILED DESCRIPTION OF THE INVENTION

In the following description, certain specific details are included to provide a thorough understanding of various disclosed embodiments. One skilled in the relevant art, however, will recognize that embodiments may be practiced without one or more of these specific details.

Unless the context requires otherwise, throughout the Specification and Claims which follow, the word "comprise" are to be construed in an open, inclusive sense, which is as "including, but not limited to".

Reference throughout this Specification to "one embodiment", or "an embodiment", or "in another embodiment", or "some embodiments", or "in some embodiments" means that a particularly referred feature, structure, or characteristic described in connection with the embodiment is included in at least one embodiment. Thus, the appearance of the phrases "in one embodiment", "in an embodiment", "in another embodiment", or "in some embodiments" in various places throughout this specification are not necessarily all referring to the same embodiment. Furthermore, the particular features, structures, or characteristics may be combined in any suitable manner in one or more embodiments.

As used herein, the term "C₁₋₈ alkyl" refers to an unbranched or branched saturated group consisting of C and H and containing 1-8 carbon atoms, including but not limited to -CH₃, -C₂H₅, n-C₃H₇, -CH(CH₃)₂, n-C₄H₉, -CH₂CH(CH₃)₂, -C(CH₃)₃, n-C₅H₁₁ and n-C₆H₁₃.

As used herein, the term "aryl C₁₋₈ alkyl" refers to a C₁₋₈ alkyl as defined above substituted with aryl, which generally is C₆-C₃₀ aryl, such as phenyl and naphthyl. Examples of aryl C₁₋₈ alkyl include but are not limited to C₆H₅CH₂- and C₆H₅CH₂CH₂-.

As used herein, the term "C₃₋₈ cycloalkyl" refers to a cyclic saturated group consisting of C and H and containing 3-8 carbon atoms, including but not limited to cyclopropyl, cyclobutyl, cyclopentyl and cyclohexyl.

In one aspect, the present application provides a process for preparing 3-mercapto propionates, comprising adding Na₂SO₃ during a reaction of H₂S with S₂R₂, wherein R= -CH₂CH₂COOR¹, R¹ is C₁₋₈ alkyl, aryl C₁₋₈ alkyl or C₃₋₈ cycloalkyl.

In some embodiments, a molar ratio of Na₂SO₃ to S₂R₂ is 1.2-2, preferably 1.28-1.5.

In some embodiments, the reaction is carried out under an atmospheric pressure.

In some embodiments, Na₂SO₃ is added in portions during the reaction. Preferably, a first portion of Na₂SO₃ is added in such an amount that Na₂SO₃ is dissolved in water at a reaction temperature to form a saturated solution of Na₂SO₃, and then the remaining portion of Na₂SO₃ is added in aliquots.

In some embodiments, a reaction time ranges from 5.5 h to 7.5 h.

In some embodiments, a reaction temperature ranges from 35°C to 65°C, preferably from 50°C to 55°C.

In some embodiments, R¹ is C₁₋₈ alkyl, including but not limited to -CH₃, -C₂H₅, n-C₃H₇, -CH(CH₃)₂, n-C₄H₉, -CH₂CH(CH₃)₂, -C(CH₃)₃, n-C₅H₁₁ and n-C₆H₁₃.

In some other embodiments, R¹ is aryl C₁₋₈ alkyl, such as C₆-C₃₀ aryl C₁₋₈ alkyl, including but not limited to C₆H₅CH₂- and C₆H₅CH₂CH₂-.

In some other embodiments, R¹ is C₃₋₈ cycloalkyl, including but not limited to cyclopropyl, cyclobutyl, cyclopentyl and cyclohexyl.

In some embodiments, the process of the present application comprises adding Na₂SO₃ during the reaction of H₂S with S₂R₂, wherein R= -CH₂CH₂COOR¹, R¹ is selected from the group consisting of -CH₃, -C₂H₅, n-C₃H₇, -CH(CH₃)₂, n-C₄H₉, -CH₂CH(CH₃)₂, -C(CH₃)₃, n-C₅H₁₁ and n-C₆H₁₃. The molar ratio of Na₂SO₃ to S₂R₂ is 1.2-2. The reaction is carried out at 35°C to 65°C under an atmospheric pressure for 5.5 h to 7.5 h, and Na₂SO₃ is added in portions during the reaction.

In some embodiments, the process of the present application comprises adding Na₂SO₃ during the reaction of H₂S with S₂R₂, wherein R= -CH₂CH₂COOR¹, R¹ is selected from the group consisting of -CH₃, -C₂H₅, n-C₃H₇, -CH(CH₃)₂, n-C₄H₉, -CH₂CH(CH₃)₂, -C(CH₃)₃, n-C₅H₁₁ and n-C₆H₁₃. The molar ratio of Na₂SO₃ to S₂R₂ is 1.28-1.5. The reaction is carried out at 35°C to 65°C under an atmospheric pressure for 5.5 h to 7.5 h, and Na₂SO₃ is added in portions during the reaction, wherein the first portion of Na₂SO₃ is added in such an amount that Na₂SO₃ is dissolved in water at the reaction temperature to form a saturated solution of Na₂SO₃, and then the remaining portion of Na₂SO₃ is added in aliquots.

In some embodiments, the process of the present application comprises adding Na₂SO₃ during the reaction of H₂S with S₂R₂, wherein R= -CH₂CH₂COOR¹, R¹ is -CH₃; the molar ratio of Na₂SO₃ to S₂R₂ is 1.28-1.5; the reaction is carried out at 35°C to 65°C under an atmospheric pressure for 5.5 h to 7.5 h; and Na₂SO₃ is added in portions during the reaction, wherein the first portion of Na₂SO₃ is added in such an amount that Na₂SO₃ is dissolved in water at the reaction temperature to form a saturated solution of Na₂SO₃, and then the remaining portion of Na₂SO₃ is added in aliquots.

The process of the present application have some advantages, such as relatively high yield of the target product, high recovery of materials, reducing the danger of reaction procedures and the like.

### EMBODIMENTS

The present application will now be described in greater detail by reference of a preparation of methyl 3-mercapto propionate.

### EXAMPLES

Reagents and their Sources
- Dimethyl: Commercially purchased, purity ≥ 98%, in
- 3,3'-dithiodipropionate: which the content of methyl 3-mercapto propionate is about 1.5%
- H₂S: Industrial products
- Na₂SO₃: Commercially purchased, purity ≥ 97%

### Example 1

To a 1000 mL flask were added 280.0 g (1.15 mol) of dimethyl 3,3'-dithiodipropionate, 106.5 g (0.845 mol) of anhydrous Na₂SO₃, and 296.0 g of water. The reaction mixture was warmed to a temperature of 50°C-55°C in water bath. H₂S (about 0.025 L/min) was introduced into the above reaction mixture for a total of 10 h. After the reaction was carried out under atmospheric pressure for 1 h (began from the introduction of H₂S), 80.0 g (0.635 mol) of Na₂SO₃ was added to the reaction mixture in 8 aliquots and each aliquot, i.e. 10.0 g of Na₂SO₃ was added every half hour. After the introduction of H₂S was stopped, the reaction temperature was maintained for 15 min. The reaction liquid was filtered under suction, and the filtrate was layered. The oil layer was washed with water, and then layered again. The resulting oil layer was distilled under reduced pressure with a circulating water pump to give methyl 3-mercapto propionate.

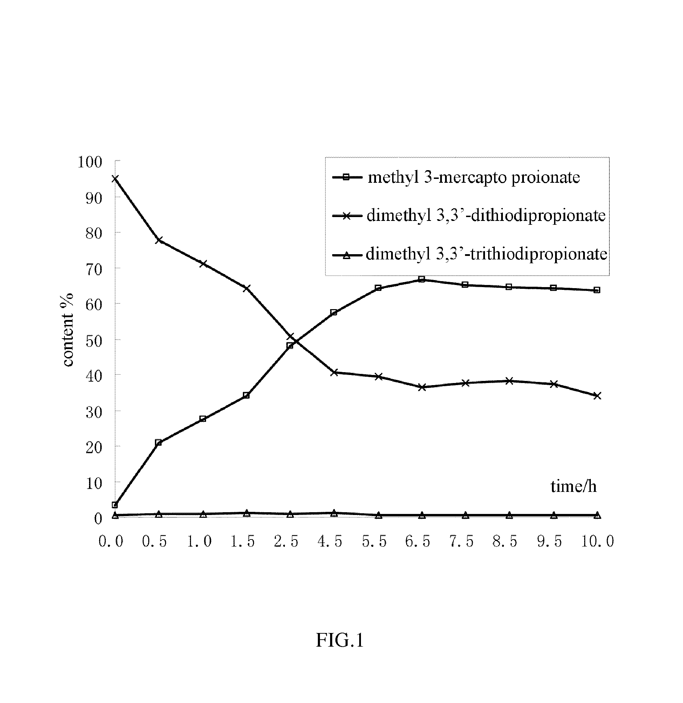

The contents of methyl 3-mercapto propionate, dimethyl 3,3'-dithiodipropionate and dimethyl 3,3'-trithiodipropionate were measured at different time points by gas chromatography and plotted. The results were shown in figure 1.

It can be seen from figure 1 that the content of target product methyl 3-mercapto propionate was relatively high, and was about 66.5% at 6.5 h.

It can also be seen from figure 1 that the content of methyl 3-mercapto propionate increased rapidly from the beginning of the reaction to the 5.5th h, and it tended to increase slowly or even decrease after the reaction was carried out for 5.5 h. Therefore, in view of cost-benefit, the reaction time ranging from 5.5 h to 7.5 h is optimal.

### Example 2

Except that the molar ratio of Na₂SO₃ to dimethyl 3,3'-dithiodipropionate (keeping the molar amount of dimethyl 3,3'-dithiodipropionate constant) and the amount of Na₂SO₃ as added each time after adding the first portion of Na₂SO₃ were different from Example 1, other reaction procedures were the same as those in Example 1, in which the above-mentioned amount of Na₂SO₃ as added each time after adding the first portion of Na₂SO₃ was determined by dividing the remaining portion of Na₂SO₃ by the total number of batches.

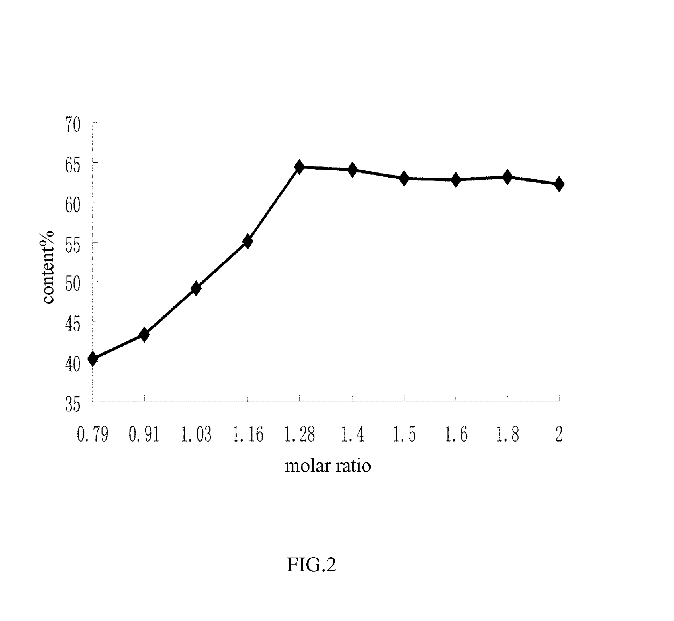

The content of methyl 3-mercapto propionate was measured at the same reaction time point and plotted. The results were shown in figure 2. In figure 2, the content of methyl 3-mercapto propionate at each molar ratio was measured when the reaction was carried out for 6h.

It can be seen from figure 2 that the content of methyl 3-mercapto propionate is highest when the molar ratio of Na₂SO₃ to dimethyl 3,3'-dithiodipropionate is from 1.2 to 2, especially from 1.28 to 1.5.

### Example 3

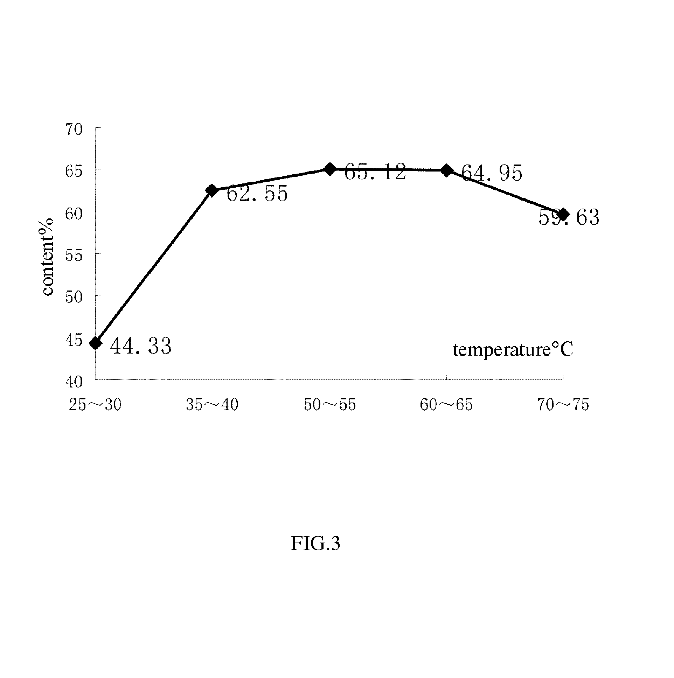

Except that the reaction temperature was different from Example 1, other reaction procedures were the same as those in Example 1. The reaction was carried out at 25°C to 30°C, 35°C to 40°C, 50°C to 55°C, 60°C to 65°C, and 70°C to 75°C, respectively. The content of methyl 3-mercapto propionate was measured at the same reaction time point and plotted. The results were shown in figure 3. In figure 3, the content of methyl 3-mercapto propionate at each reaction temperature was measured when the reaction was carried out for 6h.

It can be seen from figure 3 that the content of target product is highest at 35°C to 65°C, especially at 50°C to 55°C.

### Comparative Example 1

To a 1000 mL flask were added 280.0 g (1.15 mol) of dimethyl 3,3'-dithiodipropionate and 296.0 g of water. The reaction mixture was warmed to a temperature of 50°C-55°C in water bath. H₂S (about 0.025 L/min) was introduced into the above reaction mixture for 6 h. The reaction was carried out under atmospheric pressure. After the introduction of H₂S was stopped, the reaction temperature was maintained for 15 min. The reaction liquid was filtered under suction, and the filtrate was layered. The oil layer was washed with water and then layered again. The resulting oil layer was distilled under reduced pressure with a circulating water pump to give methyl 3-mercapto propionate.

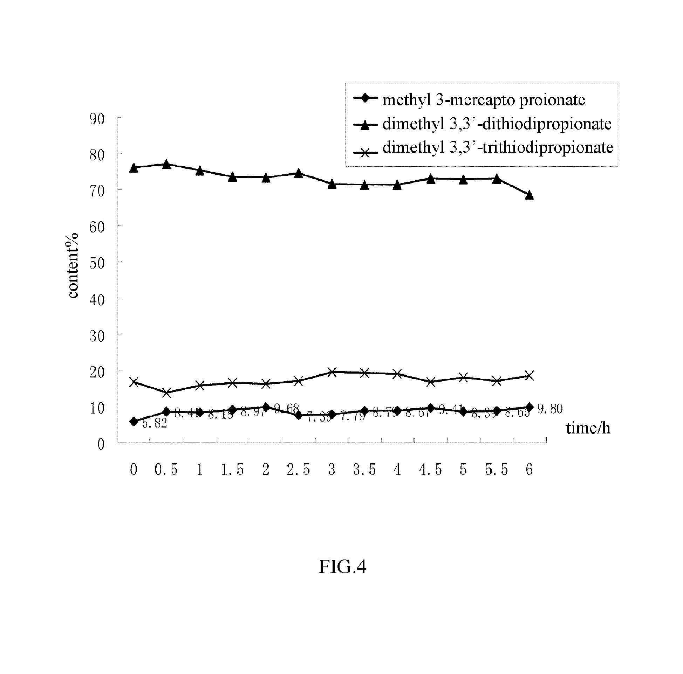

The contents of methyl 3-mercapto propionate, dimethyl 3,3'-dithiodipropionate and dimethyl 3,3'-trithiodipropionate were measured at different time points and plotted. The results were shown in figure 4.

It can be seem from the comparison between figure 1 and figure 4 that compared with the reaction without addition of Na₂SO₃, addition of Na₂SO₃ makes the reaction easier to proceed, and renders the content of the target product methyl 3-mercapto propionate relatively higher. As is shown in figure 4, the content of methyl 3-mercapto propionate varies unobviously, with the highest content being 9.8%. In contrast, figure 1 shows that when the reaction was carried out for 6.5 h, the content of methyl 3-mercapto propionate was about 66.5%.

It will be appreciated that, the description and specific examples provided in the application only serve as non-limiting illustration for understanding of the present application.

## Claims

1. A process for preparing 3-mercapto propionates, comprising adding Na₂SO₃ during a reaction of H₂S with S₂R₂, wherein R= -CH₂CH₂COOR¹, R¹ is C₁₋₈ alkyl, aryl C₁₋₈ alkyl or C₃₋₈ cycloalkyl.

2. The process of Claim 1, wherein a molar ratio of Na₂SO₃ to S₂R₂ is 1.2-2, preferably 1.28-1.5.

3. The process of Claim 1 or 2, wherein the reaction is carried out under an atmospheric pressure.

4. The process of any one of Claims 1-3, wherein Na₂SO₃ is added in portions during the reaction.

5. The process of any one of Claims 1-4, wherein a reaction time ranges from 5.5 h to 7.5 h.

6. The process of any one of Claims 1-5, wherein a reaction temperature ranges from 35°C to 65°C.

7. The process of any one of Claims 1-6, wherein R¹ is C₁₋₈ alkyl.

8. The process of Claim 7, wherein R¹ is selected from the group consisting of -CH₃, -C₂H₅, n-C₃H₇, -CH(CH₃)₂, n-C₄H₉, -CH₂CH(CH₃)₂, -C(CH₃)₃, n-C₅H₁₁ and n-C₆H₁₃.

9. The process of Claim 8, wherein R¹ is -CH₃.

10. The process of any one of Claims 1-6, wherein R¹ is selected from the group consisting of C₆H₅CH₂-, C₆H₅CH₂CH₂-, cyclopropyl, cyclobutyl, cyclopentyl and cyclohexyl.

## Patentansprüche

1. Verfahren zur Herstellung von 3-Merkaptopropionaten, umfassend das Hinzufügen von Na₂SO₃ während einer Reaktion von H₂S mit S₂R₂, wobei R=-CH₂CH₂COOR¹, R¹ C₁₋₈ Alkyl, Aryl C₁₋₈ Alkyl oder C₃₋₈ Cycloalkyl ist.

2. Verfahren nach Anspruch 1, wobei ein molares Verhältnis von Na₂SO₃ zu S₂R₂ 1,2-2, bevorzugt 1,28-1,5, ist.

3. Verfahren nach Anspruch 1 oder 2, wobei die Reaktion unter einem atmosphärischen Druck ausgeführt wird.

4. Verfahren nach einem der Ansprüche 1-3, wobei Na₂SO₃ in Portionen während der Reaktion hinzugefügt wird.

5. Verfahren nach einem der Ansprüche 1-4, wobei eine Reaktionszeit im Bereich von 5,5 Std. bis 7,5 Std. ist.

6. Verfahren nach einem der Ansprüche 1-5, wobei eine Reaktionstemperatur im Bereich von 35°C bis 65°C ist.

7. Verfahren nach einem der Ansprüche 1-6, wobei R¹ C₁₋₈ Alkyl ist.

8. Verfahren nach Anspruch 7, wobei R¹ ausgewählt ist aus der Gruppe bestehend aus -CH₃, -C₂H₅, n-C₃H₇, -CH(CH₃)₂, n-C₄H₉, -CH₂CH(CH₃)₂, - C(CH₃)₃, n-C₅H₁₁ und n-C₆H₁₃.

9. Verfahren nach Anspruch 8, wobei R¹-CH3 ist.

10. Verfahren nach einem der Ansprüche 1-6, wobei R¹ ausgewählt ist aus der Gruppe, bestehend aus C₆H₅CH₂-, C₆H₅CH₂CH₂-, Cyclopropyl, Cyclobutyl, Cyclopentyl und Cyclohexyl.

## Revendications

1. Procédé de préparation de 3-mercapto-propionates, qui comporte le fait d'ajouter du Na₂SO₃ au cours d'une réaction de H₂S avec un composé de formule S₂R₂ dans laquelle R représente un groupe de formule -CH₂CH₂COOR où R¹ représente un groupe alkyle en C₁₋₈, aryl-(alkyle en C₁₋₈) ou cycloalkyle en C₃₋₈.

2. Procédé conforme à la revendication 1, dans lequel le rapport molaire de Na₂SO₃ au composé de formule S₂R₂ vaut de 1,2 à 2, et de préférence, de 1,28 à 1,5.

3. Procédé conforme à la revendication 1 ou 2, dans lequel la réaction est effectuée sous la pression atmosphérique.

4. Procédé conforme à l'une des revendications 1 à 3, dans lequel le Na₂SO₃ est ajouté par portions au cours de la réaction.

5. Procédé conforme à l'une des revendications 1 à 4, dans lequel la durée de la réaction vaut de 5,5 à 7,5 heures.

6. Procédé conforme à l'une des revendications 1 à 5, dans lequel la température de réaction vaut de 35 à 65 °C.

7. Procédé conforme à l'une des revendications 1 à 6, dans lequel R¹ représente un groupe alkyle en C₁₋₈.

8. Procédé conforme à la revendication 7, dans lequel R¹ représente un groupe choisi dans l'ensemble formé par ceux de formules -CH₃, -C₂H₅, -n-C₃H₇, -CH(CH₃)₂, -n-C₄H₉, -CH₂CH(CH₃)₂, -C(CH₃)₃, -n-C₅H₁₁ et -n-C₆H₁₃.

9. Procédé conforme à la revendication 8, dans lequel R¹ représente un groupe de formule -CH₃.

10. Procédé conforme à l'une des revendications 1 à 6, dans lequel R¹ représente un groupe choisi dans l'ensemble formé par ceux de formules C₆H₅CH₂- et C₆H₅CH₂CH₂- et les groupes cyclopropyle, cyclobutyle, cyclopentyle et cycohexyle.
